# EUROPEAN PATENT APPLICATION

(11) **EP 4 643 802 A1**
(43) Date of publication of application: **05.11.2025**
(21) Application number: 24173442.5
(22) Date of filing: 30.04.2024
(51) Int. Cl.: A61B 34/20, A61B 34/30, A61B 90/50

(54) **ESTIMATION OF 3D POSE OF TOOLS IN MINIMALLY INVASIVE MUSCULOSKELETAL PROCEDURES**

(71) Applicant: metamorphosis GmbH, 33098 Paderborn (DE)
(72) Inventor: BLAU, Arno, 72336 Balingen (DE); SCHMIDT, Jürgen, 86497 Horgau (DE)
(74) Representative: LKGLOBAL Lorenz & Kopf Patentanwalt Attorney at Law PartG mbB

(57) **Abstract**

A device and method for assisting in endoscopic surgery is suggested, the device comprising a trocar holder and a data processing unit. The trocar holder is configured to arrange a trocar in a fixed relation to an anatomy of interest, even while the anatomy is moving. Instead of or additionally to a trocar, the trocar holder may also arrange an endoscope or an instrument in a fixed relation to an anatomy of interest. The method comprises the steps of receiving an X-ray image of the trocar and of the anatomy of interest, receiving a model of the anatomy of interest, determining a longitudinal axis of the trocar, and determining a 3D position and orientation of the trocar relative to the anatomy of interest based on the received X-ray image, on the received model of the anatomy of interest, and on the determined longitudinal axis of the trocar.

## Description

### FIELD OF INVENTION

The invention relates to computer-assisted surgery as well as to robotic-assisted surgery. The invention further relates to endoscopic surgery, 3D pose estimation of devices, mixed reality visualization of augmented information derived e.g. from a 3D dataset.

The systems and methods described herein can be applied to assist in performing steps in surgical procedures, both in minimally invasive surgery as well as musculoskeletal surgery.

### BACKGROUND OF THE INVENTION

Endoscopic spine surgery is an innovative approach that involves using small incisions and specialized tools to access and treat spinal conditions. It offers several advantages over traditional open surgery, including smaller incisions, less tissue damage, reduced blood loss, faster recovery times, and lower risk of complications.

Some of the specific applications of endoscopic spine surgery include:
Discectomy: Endoscopic discectomy is often used to treat herniated discs in the spine. The surgeon uses an endoscope to visualize the affected disc and remove the herniated portion, relieving pressure on the spinal nerves and reducing pain.

Foraminotomy: This procedure involves enlarging the neural foramen, the passageway through which spinal nerves exit the spinal canal. Endoscopic foraminotomy can relieve nerve compression caused by conditions such as foraminal stenosis or bone spurs.

Facet Joint Surgery: Endoscopic procedures can also address issues with the facet joints, which are small joints located between each vertebra. Endoscopic facet rhizotomy or denervation involves selectively disrupting the nerves that transmit pain signals from the facet joints, providing relief for chronic back pain.

Laminectomy/Laminotomy: Endoscopic laminectomy or laminotomy involves removing part of the lamina, the bony arch of the vertebra, to relieve pressure on the spinal cord or nerves. This can be used to treat conditions such as spinal stenosis.

Spinal Fusion: While less common, endoscopic techniques can also be used in spinal fusion procedures. Endoscopic fusion involves placing bone graft material between vertebrae to promote fusion, using specialized instruments inserted through small incisions.

Minimally Invasive Surgery (MIS): Endoscopic spine surgery is part of the broader trend toward minimally invasive spine surgery. MIS techniques aim to achieve the same surgical goals as traditional open surgery while minimizing tissue damage and speeding up recovery. Endoscopic spine surgery is continually evolving, with ongoing advancements in technology and techniques.

US 8,114,085 B2 describes implementations for a percutaneous spinal registration-and-access tool for minimally invasive spinal surgery.

For the surgeon, it is difficult to orient in the patient anatomy especially if there is only one entry path for instruments and endoscope and thus the endoscopic image only depicts a very narrow part of the anatomy which makes it difficult to identify anatomical structures.

### SUMMARY OF THE INVENTION

At least the one or the other of the mentioned problems are mitigated or solved by the subject matter according to each of the independent claims. Further embodiments are described in the respective dependent claims.

The invention is intended to determine a 3D pose, meaning the 3D Position and orientation of at least an endoscope, but potentially also a trocar, a knife, a pliers or any other minimal invasively used instrument relative to an anatomy, while the anatomy could be a bone, an intervertebral disc, or any other part of an anatomy.

According to an aspect of the invention, the 3D position and orientation of the endoscope relative to an anatomy of interest may be determined based on a video image provided by the endoscope and on a model of the anatomy of interest which is visible in the video image.

According to another aspect of the invention, a device is provided for assisting in endoscopic surgery comprises a trocar holder and a data processing unit. The trocar holder is configured to arrange a trocar in a fixed relation to an anatomy of interest, even while the anatomy is moving. Instead of or additionally to a trocar, the trocar holder may also arrange an endoscope or an instrument in a fixed relation to an anatomy of interest.

The data processing unit is particularly configured to receive an X-ray image of the trocar and of the anatomy of interest, to receive a model of the anatomy of interest, and to determine a 3D position and orientation of the trocar relative to the anatomy of interest based on the received X-ray image and on the received model of the anatomy of interest.

The data processor may further be configured to determine a geometrical aspect of the trocar and/or of the endoscope as visible in the X-ray projection image. The geometrical aspect of the trocar and/or the endoscope may be a part of an outline or contour of the projection of the same. A part of the outline may be a line at one side of a shaft or a pair of lines at opposite sides of the shaft, with the sides extending in a longitudinal direction of the shaft. Further, the geometrical aspect may be a distal tip of the trocar and/or of the endoscope. The gepmetrical aspect may also be a longitudinal axis of the shaft. When determining the 3D position and orientation of the trocar and/or of the endoscope relative to the anatomy of interest, the data processor may be configured to take into account the determined geometrical aspect.

A method according to an embodiment for determining the position of a minimally invasive device inserted in a patient's body may comprise the following steps of obtaining a 3D model of at least a part of a bony anatomy of a patient, optionally obtaining a 3D model of the device to be inserted in the anatomy, fixating the device to the anatomy (e.g., to a pedicle of a vertebra), determine the relative position of the device to the vertebra based on an X-ray image, wherein the device is connected to a trocar holder, inserting the device into the patient (with or without using a trocar), optionally acquiring an X-ray image, and determining the relative position of at least an axis of the device to the anatomy.

In other words, a trocar holder may be configured to be fixedly arranged at the moving anatomy so that the trocar holder moves together with the moving anatomy. For example, the anatomy of interest may be a vertebra of a spine and the trocar holder may be fixedly arranged at the vertebra by means of a pedicle screw. In such a situation, the 3D position and orientation of the trocar relative to the anatomy of interest may be determined based on a determination of a 3D position and orientation of the pedicle screw relative to the anatomy of interest.

Otherwise, the trocar holder may be a robotic trocar holder configured to move the trocar holder together with the moving anatomy so as to keep the trocar holder in the fixed relation to the anatomy of interest.

When utilizing a robotic trocar holder, the relative position of the trocar holder to the anatomy of interest may automatically be adjusted to the needs, with the data processing unit identifying the steps of the surgical procedure and suggesting adjustments depending on the expected next step.

According to an embodiment, a tracking device is provided being configured to track the 3D position and orientation of at least one of the anatomy, the trocar holder, the trocar, an endoscope, and an instrument. The tracking device may perform a real time tracking in order to determine changes of the relative position of the at least the axis of the trocar or any other device relative to the anatomy. The tracking device may determine the relative position of at least an axis of the trocar or other device by at least one of: (i) a real time tracking provided by sensory of a robot adjusting from a default (known) relative position between trocar holder and the device to be inserted, (ii) a real time tracking provided by at least one camera seeing the trocar holder, (iii) a real time tracking provided by at least one camera seeing at least part of the trocar or an attachment to the trocar, (iv) acquiring an X-ray image depicting at least part of the device to be inserted and at least one of the trocar holder, the trocar, the attachment to the trocar.

An endoscope may be configured to be arranged within a trocar, wherein the 3D position and orientation of the trocar relative to the anatomy of interest may be determined together with a 3D position and orientation of the endoscope relative to the anatomy of interest. The endoscope may be configured to provide a video signal of the anatomy of interest. According to an embodiment, the 3D position and orientation of the endoscope relative to the anatomy of interest may be determined, inter alia, based on the video signal provided by the endoscope.

According to an aspect of the disclosure, a device for augmenting image information of an endoscope is provided, wherein the endoscope comprises a longitudinal axis, a distal tip and a viewing direction away from the distal tip, and wherein the endoscope is configured to provide video images of at least a part of an outer surface of an anatomy of interest in front of the distal tip. The device comprises a data processing unit in accordance with an embodiment, wherein the data processing unit may be configured to determine a 3D position and orientation of the endoscope relative to the anatomy of interest, to receive a model of the anatomy of interest, and to augment a video image provided by the endoscope with information of the model of the anatomy of interest.

It is noted that the term "video image" in the context of this disclosure encompasses single images as well as a sequence of images. That is, an endoscope may provide a single image like a photograph, wherein said image may be processed so as to be augmented in accordance with the present disclosure. Further, the endoscope may provide a sequence of images which can be viewed like a video. The term "video image" is intended to distinguish the kind of the image from, e.g. an X-ray or MRT image.

The data processing unit may also be configured to receive a video signal of the endoscope and to classify structures of the anatomy of interest based on the video signal and to augment the visibility of the classified structures when providing an augmented video image based on the video signal.

Furthermore, the data processing unit may be configured to receive a video signal of the endoscope and to augment the video signal with information from the model of the anatomy of interest when providing an augmented video image based on the video signal. As an example, an MRT scan or a CT scan may be fused to the model so as to provide an augmented video image. The model of the anatomy of interest may be based on an anatomy scan.

According to an embodiment, a deep neural net may be applied when processing the video signal of the endoscope. For example, the deep neural net may be applied to classify anatomical structures visible in the video image provided by the endoscope, and/or the deep neural net may be applied to augment the video image provided by the endoscope by outlining anatomical structures visible in the video image.

It will be understood that it may be advantageous to know the spatial position of the distal end of the endoscope including, e.g., a camera, relative to the anatomy which is viewed by the camera of the endoscope, so as to provide an augmented video image. The 3D position and orientation of the endoscope relative to the anatomy of interest may be determined based on the viewing direction of the endoscope and a distance between the distal tip of the endoscope and the outer surface of the anatomy of interest visible in the video image provided by the endoscope.

An algorithm may be able to generate a virtual endoscopic image or a plurality of virtual endoscopic images based on a 3D model and a certain 3D position and 3D orientation of the endoscope (i.e. its image plane) relative to the 3D model. A virtually generated endoscopic image may be generated based on the 3D data of an anatomy, existing information of a 3D position and 3D orientation of a real endoscope (i.e. its image plane) relative to the anatomy and may be compared with a real endoscopic image that is acquired from a current position of the endoscope relative to the anatomy. This comparison may be also based on detection and/or labelling of certain anatomical structures in the real endoscopic image, which may be performed e.g. by a neural net, and comparing these anatomical structures with the structures that may be detected/labelled in the 3D data and thus depicted and/or labelled in the virtual endoscopic image.

The comparison information may be utilized to determine a grade of similarity of the image content of the real and the virtual endoscopic image, in order to validate or potentially correct the existing information of a 3D position and 3D orientation of a real endoscope (i.e. its image plane) relative to the anatomy.

For a determination of a 3D position and orientation of an endoscope relative to the anatomy, a plurality of virtual video images of the endoscope may be generated viewed from different viewing angles and viewing positions onto the anatomy of interest and each of the virtual video images may be compared with the video image provided by the endoscope. The determination of the 3D position and orientation of the endoscope relative to the anatomy is, thus, based on determining one virtual video image out of the plurality of virtual video images, with the one virtual video image having a viewing angle and a viewing position onto the anatomy of interest which is best matching the video image provided by the endoscope.

The virtual endoscopic image or images may be generated during surgery or before surgery based on a planning procedure where the surgeon plans the desired position of the endoscope.

Based on the planned desired position of the endoscope, an algorithm may propose an ideal position for the screw element of the trocar holder and may additionally determine a default trocar holder assembly. This default trocar holder assembly, i.e. the way movable parts of the trocar holder need to be adjusted to reach the default trocar holder assembly may then be adjusted preoperatively or intraoperatively before placing the trocar relative to the anatomy.

If the screw part of the trocar holder is positioned in the anatomy as planned and the default trocar holder assembly is attached to the screw part, this would enable a trocar position and orientation relative to the anatomy as planned. A deviation of the position of the screw part compared with the planned position may be detected based on an X-ray image and an algorithm detecting the screw in the X-ray image. The 3D value (six degrees of freedom) may be taken into account for providing the navigational information i.e. the 3D position and 3D orientation of the trocar relative to the anatomy.

Another aspect of the disclosure may be to determine the relative 3D position and 3D orientation of at least another object visible in an endoscopic image relative to an anatomy. This object may be e.g. pliers, knife, drill or an implant. This object may be steered manually or by a robot. In case of a robot steering this object, the robot may use navigational information for an automatic or semi-automatic positioning (e.g. so called no fly zone positioning) or it could be steered manually. In either case it may be beneficial to exactly know the relative 3D position and 3D orientation of the object relative to the anatomy or relative to other objects that may be visible in the endoscopic image.

This determination of the relative 3D position and 3D orientation of at least another object may be based on the 3D model of the at least another object and generating a plurality of virtual endoscopic images from different viewing angles and distances of at least this object. Based on image processing of the real endoscopic image depicting at least this object and comparing content of the real endoscopic image with the plurality of virtually generated endoscopic images, an algorithm selects the one virtually generated endoscopic image that is the best match to the real endoscopic image. This may be based on a shape and/or appearance matching.

The determination of the relative 3D position and 3D orientation of at least another object may additionally or alternatively be based on acquisition of an X-ray image depicting the anatomy and at least the other object and image processing of the X-ray image.

This determination of the relative 3D position and 3D orientation of at least another object may additionally or alternatively be based on navigational information based on the 3D model of the object and e.g. on at least a head mounted camera or any other camera able to track a part of the object that may be visible to the camera.

Alternatively or additionally, the 3D position and orientation of the endoscope relative to the anatomy of interest may be determined based on an X-ray image of at least a part of the endoscope including the distal tip and of the anatomy of interest in front of the distal tip. The data processing unit may be configured to receive the X-ray image of the endoscope and of the anatomy of interest, to determine a longitudinal axis of the endoscope, and to determine the 3D position and orientation of the endoscope relative to the anatomy of interest based on the X-ray image, on the model of the anatomy of interest, and on the determined longitudinal axis of the endoscope.

The device may further comprise a display for visualizing the augmented video image, wherein the display may be a head mounted display allowing a visualization of 3D information by the augmented video image, or a display of a monitor.

Most of the above-mentioned aspects may be implemented as a computer program product executable on a data processing unit of a device for assisting in endoscopic surgery. The computer program product, thus, comprises sets of instructions which when executed by the data processing unit causing the device to receive an X-ray image of a trocar and of an anatomy of interest, wherein a trocar holder is configured to arrange the trocar in a fixed relation to the anatomy of interest, while the anatomy is moving, to receive a model of the anatomy of interest, to determine a longitudinal axis of the trocar, and to determine a 3D position and orientation of the trocar relative to the anatomy of interest based on the received X-ray image, on the received model of the anatomy of interest, and on the determined longitudinal axis of the trocar.

The computer program product may also include sets of instructions which when executed by the data processing unit causing the device to receive a video signal from an endoscope and to determine a 3D position and orientation of the endoscope relative to the anatomy of interest based on the video signal provided by the endoscope. Furthermore, the data processing unit may cause the device to provide an augmented video image based on the video signal.

As a skilled person will appreciate, the computer program product may be stored on a non-transitory computer-readable medium.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an embodiment including a trocar holder fixed to a patient.
Fig. 2 schematically shows an embodiment including a robotic trocar holder.
Fig. 3 is a flow chart illustrating steps of a method in accordance with the disclosure.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

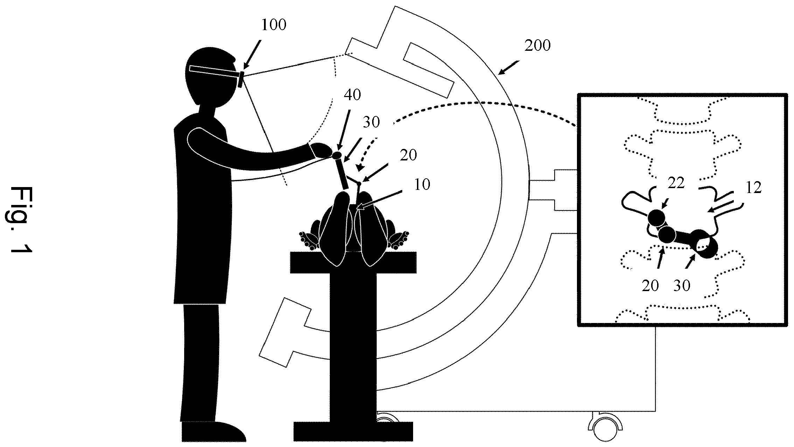

Fig. 1 schematically shows a situation in which a patient 10 is treated in a surgical intervention by a surgeon. The patient is laying on an operation table with its back facing upwards so that the surgeon can access the spine of the patient. For the treatment of at least one vertebra of the spine, a trocar holder 20 is utilized by means of which a trocar 30 can be fixedly arranged relative to the spine. Through the trocar, an endoscope 40 is inserted, wherein a distal end of at least the endoscope is intended to extend into the body of the patient in the vicinity of the spine allowing the surgeon to minimally invasively access a vertebra of the spine.

Having in mind that the patient is breathing and, thus, that the spine is moving accordingly, it may be seen as a first aim to provide a possibility to keep an endoscope stable and fix relative to the moving spine so that the video image of the endoscope appears still on a monitor or display. According to a first embodiment, it is suggested to fixedly attach a trocar holder to the moving anatomy, e.g., to one of the vertebras 12.

That first embodiment is illustrated in fig. 1 depicting a plane view from above onto the spine showing several vertebras 12. In the detailed plane view the trocar holder 20 is visible together with the trocar 30. In this particular example, the trocar holder 20 is attached to a vertebra 12 by a pedicle screw 22. As may be understood, the plane view onto the vertebras may be an X-ray image generated by the C-arm based X-ray imaging device 200 as in fig. 1.

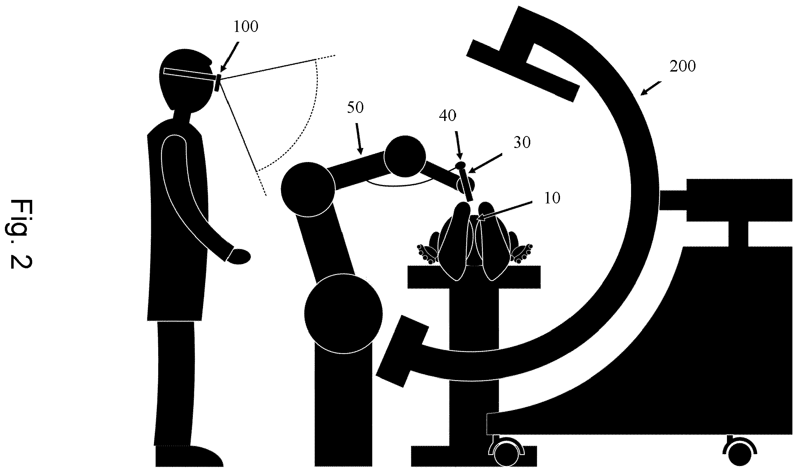

According to a second embodiment, it is suggested to hold the endoscope with a robotic arm 50 (shown in fig. 2) and a real time tracking system tracks the movements of the spine, wherein the robotic arm is configured to move the endoscope following the tracked movements of the spine. That second embodiment is illustrated in fig. 2. As an example, a head mounted display 100 is depicted providing a tracking functionality. It will be understood that not only a movement of the anatomy may be tracked by the tracking system but also any movement of the endoscope or of any instrument used for treatment of the patient.

The head mounted display 100 as depicted in both fig. 1 and fig. 2 may further provide the functionality of a display showing the video images of the endoscope alone or in combination with further information. That information may provide guidance for the surgeon through the interventional steps of the treatment.

For example, a 3D model or representation of at least the considered anatomy may be utilized to provide an overview over the anatomy although the video image of the endoscope has only a limited field of view. Such a model may either be preoperatively acquired e.g. by means of a CT or MRT scan, it may also be intraoperatively acquired by a 3D scanning device like a C-arm or an O-arm, it may also be computed based on a plurality of two-dimensional images, intra-op C-arm images and some a-priori knowledge like a statistical shape model that mathematically describes at least the anatomy of interest whereas 2D images are used to refine the 3D model to better fit the actual patient's anatomy.

A fusion or registration of different imaging modalities may also be applied to provide and/or enhance information, e.g. an existing MRT 3D model may be fused to a CT model, and/or an intraoperatively acquired 2D image may be used to refine at least one previously acquired 3D model, which may be necessary to enhance accuracy of the 3D representation of the patient's anatomy due to intraoperative movement in between some aspects of the patient's anatomy. An intraoperative 2D image may be e.g. an X-ray image or an endoscopic image. This intraoperatively intra-anatomical movement may be e.g. a relative movement of vertebrae that may occur e.g. due to repositioning of a patient, or due to a surgical step that changes the relative positions of vertebrae. An intra-anatomical movement may also occur e.g. due a resection, an osteotomy, a drilling, an implantation.

Even if there may be no intra-anatomical movement, in a surgical procedure there always is a movement between surgical instruments or implants and the anatomy. After an initial determination of a 3D position and 3D orientation between a surgical tool relative to an anatomy, this may be advantageous to the surgeon to track this movement by means of knowing and/or displaying this changed 3D position and 3D orientation between a surgical tool relative to the anatomy, also it may be advantageous to calculate certain values like distances and angles based on this tracking and the initial determination of 3D position and 3D orientation between a surgical tool relative to the anatomy.

There are different methods to determine the initial 3D position and 3D orientation between a surgical tool relative to an anatomy. This determination may often be also called registration. This registration is described in many ways in the literature of intraoperative computer navigation and e.g. may be based on intraoperative 3D imaging, the tracking of the movement may be derived from a 3D camera observing positions of trackers attached to anatomy and instruments. But these methods add significant effort during the surgical procedure and may also be not reliable enough.

Both WO 2021/122804 A1 (Blau et al.) and WO 2022/243316 A1 (Blau et al.) teach how to reliably determine 3D position and 3D orientation between a surgical tool relative to an anatomy based on single intraoperatively acquired 2D X-ray images and a-priori knowledge of 3D position of a certain geometrical aspect of the instrument relative to the anatomy (e.g. a tip of a drill sitting on an anatomy like touching a surface of a bone). But this a-priori knowledge may not always be available. This may especially be the case e.g. in endoscopy supported surgical procedures where there is need to determine the 3D position and 3D orientation between an endoscopic image (i.e. the imaging plane of the endoscope) relative to an anatomy without having the endoscope touching a surface of an anatomy.

The endoscope may be inserted in a trocar and its position relative to the trocar may be known (e.g. due to mechanical stop in the trocar). At least in such cases it may be advantageous to determine the position and track the movement of the trocar relative to the anatomy. This may be determined by applying above procedures and methods, but like the endoscope, the trocar also may not be positioned in a way the a-priori information can be used to determine its position based on a single intraoperative X-ray as taught by Blau et al.

The disclosure is intended to determine a 3D position and orientation of at least an endoscope, but potentially also a trocar, a knife, a pliar or any other minimally invasively used instrument relative to an anatomy, while the anatomy may be moving. The anatomy could be a bone, an intervertebral disc, or any other part of an anatomy. It may be the case that neither the trocar nor the endoscope touch the surface of the anatomy, or it is not known whether it touches it, or if it is known, there is no 3D model available of the surface of this part of the anatomy, or there is need for an additional way of determination of the relative 3D position and 3D orientation between the anatomy and the trocar and/or the endoscope.

One aspect of the disclosure may be inserting a supporting instrument of sufficiently known geometry in another and stable part of the anatomy (e.g. in a bony area like a pedicle of a vertebra). The relative 3D position and 3D orientation between the supporting instrument and the anatomy may be known. This may be done e.g. as taught by WO 2023/110124 A1 and/or WO 2023/247328 A1. At least part of this supporting instrument may also be a temporary or permanent implant.

Further, the endoscopic image may be utilized for several different purposes. It may provide visual information to the surgeon. The visual information may also be processed e.g. by means of a deep neural net in order to e.g. determine, classify and/or depict anatomical structures and/or instruments/tools/implants visible in the endoscopic image to generate labeled content. The content may be utilized to further enhance the 3D model.

A further aspect of the disclosure may be to utilize the visual information provided by the endoscope potentially in combination with one or more determined/classified depicted anatomical structures to either determine the relative 3D position and 3D orientation between the endoscope (i.e. its imaging plane) and the anatomy. Additionally or alternatively, the visual information of the endoscope is utilized to provide information on which the tracking of the movement of the anatomy is based.

One embodiment of the disclosure suggests determining the initial 3D position and 3D orientation between the anatomy and the endoscope and/or trocar by any method described above not using the endoscopic image and track any following movement of the anatomy or instrument by utilizing at least the endoscopic image.

Another aspect of the disclosure is to augment a two-dimensional image provided by an endoscope with information derived e.g. from 3D data or 2D X-ray image in order to virtually broaden the field of view of the endoscope.

Another aspect of the disclosure is providing an immersive visualization of the anatomy by above described determining and tracking the relative 3D position and 3D orientation of the endoscopic image plane and the anatomy and virtually augment it by displaying structures of the anatomy.

Another aspect of the disclosure is to generate virtual endoscopic image area based on the 3D data surrounding the real endoscopic field of view.

A user may virtually change the endoscopic image plane, change the field of view and/or viewing angle or viewing direction.

Virtual flight through the anatomy is also envisaged, e.g. starting at a current position of the actual imaging plane of the endoscopic image and utilizing all information of the enhanced 3D model.

Another aspect of the disclosure may be at least one robotic arm supporting the procedure. One robotic arm may hold the trocar and/or the endoscope and keep its relative position to the anatomy although the anatomy is moving by tracking the movement, e.g. provided by a camera that may be attached to the same or to another robotic arm. Additionally or alternatively, the tracking of the movement of the anatomy is provided by the endoscopic image.

Further, a mechanical assembly with at least one movable and fixable joint holding the trocar may be rigidly attached in a default position relative to the supporting instrument.

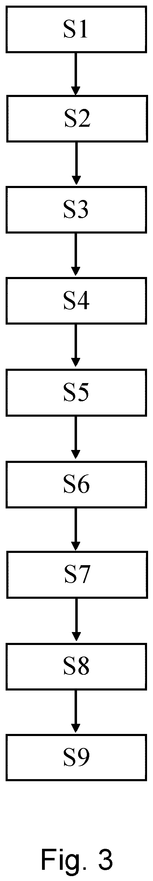

With reference to fig. 3, a method according to an embodiment for assisting in endoscopic surgery is described, the method comprise the steps of
S 1 providing a trocar holder being configured to arrange a trocar in a fixed relation to an anatomy of interest, while the anatomy is moving,
S2 receiving an X-ray image of the trocar and of the anatomy of interest,
S3 receiving a model of the anatomy of interest,
S4 determining a longitudinal axis of the trocar, and
S5 determining a 3D position and orientation of the trocar relative to the anatomy of interest based on the received X-ray image, on the received model of the anatomy of interest, and on the determined longitudinal axis of the trocar.

According to an embodiment, determining the 3D position and orientation of the trocar relative to the anatomy of interest (S5) may include determining a 3D position and orientation of an endoscope relative to the anatomy of interest, with the endoscope being placed within the trocar.

Additionally, determining the 3D position and orientation of the endoscope relative to the anatomy of interest (S5) may be based on a video signal provided by the endoscope.

The method may also comprise a step (previous to step S1) of fixedly arranging the trocar holder at the moving anatomy so that the trocar holder moves together with the moving anatomy. Where the anatomy of interest comprises a vertebra of a spine and where the trocar holder is fixedly arranged at the vertebra by means of a pedicle screw, determining the 3D position and orientation of the trocar relative to the anatomy of interest may be based on a determination of a 3D position and orientation of the pedicle screw relative to the anatomy of interest.

According to an embodiment, the trocar holder may be a robotic trocar holder configured to move the trocar holder together with the moving anatomy so as to keep the trocar holder in the fixed relation to the anatomy of interest.

The method may further comprise step S6 of tracking the 3D position and orientation of at least one out of the group consisting of the anatomy, the trocar holder, the trocar, and the endoscope.

The method may also comprise step S7 of receiving a video signal of the endoscope and to classify structures of the anatomy of interest based on the video signal and to augment the visibility of the classified structures when providing an augmented video image based on the video signal.

The method may also comprise step S8 of receiving a video signal of the endoscope and to augment the video signal with information from the model of the anatomy of interest when providing an augmented video image based on the video signal.

It is noted that a deep neural net may be applied when processing the video signal of the endoscope.

Finally, the method may comprise step S9 of displaying the augmented video image.

Before performing the method, some aspects may be planned. In a preoperative planning based on 3D image data of the anatomy of interest, a position of the pedicle screw element of the trocar-holder may be planned, a desired position of the trocar and or of the endoscope may be planned based on which an algorithm determines the shape of the trocar holder connecting the pedicle screw element with the trocar or endoscope. This case specific trocar-holder may be 3D printed and may be used intraoperatively as a sterile single use device for positioning of the trocar while having an initial determination of the relative 3D position and orientation between trocar/endoscope and anatomy.

Such an initial determination of the relative 3D position and orientation between trocar or endoscope and anatomy may be refined based on the actual drilling of the pedicle hole of the pedicle screw element and or based on the inserted pedicle screw element which may be assessed by acquiring and processing an X-ray image of the pedicle screw element in the anatomy.

Other ways of an initial determination of the relative 3D position and orientation between trocar/endoscope and anatomy may be, a surgeon positioning the trocar or endoscope as planned, a navigated robot positioning the trocar or endoscope as planned, or an X-ray of the endoscope is acquired and utilized.

Based on image processing of the endoscopic image, e.g. by utilizing a deep neural net detecting and/or classifying anatomical structures in the endoscopic image, and/or may be comparing the endoscopic image with a plurality of virtual generated endoscopic images from different viewing angles and viewing positions, an additional refinement that may be updated in real time may be processed.

While the method with reference to fig. 3 is described more generally, steps of an exemplary method are described for further understanding of the present disclosure in more detail.
1. Preoperative planning is performed of at least one drilling trajectory in a pedicle designated for attaching the trocar holder to a vertebra, optionally, including incision point and or incision trajectory;
2. Optional: Preoperative planning is performed of one or more resection volume(s) in a preoperative 3D data like CT or MRT;
3. Optional: One or more trocar position(s) or only its trajectory(ies) are planned relative to 3D data;
4. Optional: Ideal C-arm position or only C-arm viewing direction is planned in order to prevent ambiguities and mechanical collisions, e.g. with surgeon's head. This may be supported by automated algorithms, optionally DNN based;
5. In case of MRT and CT: 3D/3D match is calculated in order to provide MRT 3D data for intraoperative navigation based on an intraoperative matching of 2D X-ray and preoperative CT data. Alternative: If there is only MRT available, a 3D/3D matching of intraoperative 3D data is performed (e.g. acquired by a 3D C-arm or O-arm), and then 2D (C-arm X-ray image) matching is performed with previously acquired intraoperative 3D data (already matched to MRT data);
6. A C-arm image is acquired from oblique lateral or oblique AP viewing direction. The advantage of this C-arm position is, that the image intensifier of the C-arm is not colliding with the surgeon's head position. Having this flexibility is further beneficial e.g. in case of a strong kyphosis (or lordosis): As there is no need of a true AP position, there is high flexibility how to position the C-arm to prevent collision conflicts. Also the position of the C-arm cart on the floor is variable. In case there is need for a rotation of the C-arm, this can be done without risking collision with the surgeon, too. As the registration procedure is designed to work with single C-arm image, it is possible to quickly achieve guidance for performing an incision as well as the positioning of the drill bit on the desired entry point (e.g. on the pedicle).
7. The system matches the C-arm based X-ray image to 3D data and thus calculates the relative position of C-arm to the anatomy.
8. If needed: The system (iteratively) guides the surgeon (or instructs a robotic C-arm) how to reposition the C-arm in order to achieve the planned position of the C-arm, or if there is no planned position available, calculates if there is a need to reposition the C-arm, e.g. due to possible ambiguities resulting from combination of imaging direction and entry point on bone. If a mixed reality (MR) device is used, it may identify components of C-arm used in order to instruct, e.g. which lever to pull in order to achieve desired movement of C-arm.
9. As soon as there is a C-arm image available from a desired or sufficient imaging direction, the system again calculates a match of the 3D data with the 2D X-ray image and displays virtual augmented information either superimposed to the X-ray image and/or superimposed to the detected and tracked instrument (e.g. scalpel), which tracking and displaying may be performed by an MR device.
10. In case of a real time tracking of the scalpel, the incision is guided in real time. Otherwise, there may be a static guidance solely to find the right incision point.
11. As soon as the incision is performed, the drill is inserted with its tip of the drill bit touching the bone surface and another X-ray (C-arm image) may be acquired. If the tip is not yet close enough to the planned entry point, the system provides either static instructions on how to reposition the tip or real time instructions in case the drilling machine is tracked (e.g. by the head mounted MR device) until there is either an X-ray image available showing a sufficiently close position between the actual tip and the planned entry point and/or until the real time tracking detects this.
12. Based on the assumption that the tip touches the surface sufficiently close to the planned entry point, the system calculates the relative pose of the drill and the anatomy and provides guidance in order to achieve a sufficiently aligned drill with planned drilling trajectory. This can be an iterative procedure based on acquired X-ray images (no changes in C-arm position needed) or a real time tracking (e.g. in form of arrows depicting a needed angular correction, if any). If a robot is used for the procedure, there is no need for displaying all the navigational information - the robot could directly follow the instruction.
13. If drilling has started, the real time tracking may provide updated information whether the drilling trajectory is still in line, in addition or alternatively, another X-ray image may be acquired in order to determine the relative pose between drill and anatomy/planned drilling trajectory.
14. A pedicle screw-trocar holder is inserted in the drilled pedicle hole. The system saves its 3D position in the vertebra either based on the drilling or, if the pedicle screw is part of the trocar holder, it also tracks or depicts it in another X-ray image acquired by the C-arm, based on its detected position in the bone.
15. The trocar holder may consist of movable joints:
   a. optional, the joints could be preoperatively adjusted and fixed (relative position of trocar and pedicle screw part), and thus the position of the trocar may be known to the system after an assembly of the trocar to a pedicle screw element of the trocar holder, the deviating position from the originally planned position of the pedicle screw may be incorporated in the positioning determination thereof.
   b. a robot may move the trocar with the movable part of the trocar holder from its previously known default position to a new position relative to the pedicle screw element so as to meet the position as planned.
   c. a real time tracking system, e.g. camera(s) from a head mounted MR device, may track the changes in position of the movable trocar (-holder) to guide to the desired position as planned, alternatively in case no desired trocar (-holder) position is planned, simply the positioning information of the current position relative to the anatomy (or if available relative to the planned resection area) while moving is displayed.
   d. in addition or alternatively to the aforementioned, an X-ray image may be acquired after moving the trocar (-holder) to the presumably desired position and the X-ray image validates this position.
   e. in addition, a further X-ray image from a different imaging direction may be acquired to obtain even more certainty in calculating its position, even though the trocar's tip is not touching bony structure.
   f. in addition or alternatively, the trocar may be positioned with its tip on a bony structure, an X-ray image may be acquired and the trocar's pose relative to the anatomy is calculated, or if its tip is not yet close enough to the planned tip position, the system provides instructions to move it accordingly (e.g. based on superposition of arrows/measurements with directions).
   g. the trocar holder may be manually adjusted.
16. When the system assumes that the trocar is placed at a desired position relative to anatomy (e.g. a vertebra), the position of an (in the trocar) inserted endoscope is displayed via 3D navigational information by the system, the insertion depth of the endoscope may either have a default position within the trocar or is tracked via a camera.
17. Optional: The position of the endoscopic imaging plane relative to the anatomy is validated and may be corrected by applying image processing algorithms comparing the endoscopic image as depicted with a virtual endoscopic image generated based on the navigational information and the 3D data.
18. Optional: Automatic detection of depicted anatomical structures in an endoscopic image whose determination may be (also) supported by a deep neural net (DNN).
19. Optional: the automatic detection is supported by navigational information of the position of the endoscope and the 3D data.
20. Optional: The position of the endoscopic imaging plane relative to the anatomy is validated and may be corrected by algorithms comparing the detected anatomical structures in the endoscopic image with the anatomical structures derived from the 3D data in combination with the navigational information.
21. Automatic virtual enhancement of the endoscopic image is provided around the actual visible endoscopic viewing area by means of the navigational information and the 3D data
   a. as (possibly semitransparent) overlay imaging plane derived from 3D data;
   b. as virtually generated endoscopic image enhancing the actual endoscopic image in order to get an immersive impression.

It will be understood that the steps described with respect to both methods above are steps which might be differentiated or divided into sub-steps. Furthermore, there might be also sub-steps between the described steps. It will also be understood that only part of the whole method may constitute the invention, i.e. steps may be omitted.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device for assisting in endoscopic surgery, the device comprising
a trocar holder, and
a data processing unit,
wherein the trocar holder is configured to arrange a trocar in a fixed relation to an anatomy of interest, while the anatomy is moving, and
wherein the data processing unit is configured
to receive an X-ray image of the trocar and of the anatomy of interest,
to receive a model of the anatomy of interest, and
to determine a 3D position and orientation of the trocar relative to the anatomy of interest based on the received X-ray image and on the received model of the anatomy of interest.

2. The device of claim 1, further comprising an endoscope, wherein the endoscope is configured to be arranged within the trocar, wherein determining the 3D position and orientation of the trocar relative to the anatomy of interest includes determining a 3D position and orientation of the endoscope relative to the anatomy of interest.

3. The device of claim 2, wherein the endoscope is configured to provide a video signal of the anatomy of interest, wherein determining the 3D position and orientation of the endoscope relative to the anatomy of interest is based on the video signal provided by the endoscope.

4. The device of any one of claims 1 to 3, wherein the trocar holder is configured to be fixedly arranged at the moving anatomy so that the trocar holder moves together with the moving anatomy.

5. The device of claim 4, wherein the anatomy of interest comprises a vertebra of a spine and wherein the trocar holder is fixedly arranged at the vertebra by means of a pedicle screw, wherein determining the 3D position and orientation of the trocar relative to the anatomy of interest is based on a determination of a 3D position and orientation of the pedicle screw relative to the anatomy of interest.

6. The device of any one of claims 1 to 5, wherein the trocar holder is a robotic trocar holder configured to move the trocar holder together with the moving anatomy so as to keep the trocar holder in the fixed relation to the anatomy of interest.

7. The device of any one of claims 1 to 6, further comprising a tracking device configured to track the 3D position and orientation of at least one out of the group consisting of the anatomy, the trocar holder, the trocar, and the endoscope.

8. The device of claim 2, wherein the data processing unit is configured to receive the video signal of the endoscope and to classify structures of the anatomy of interest based on the video signal and to augment the visibility of the classified structures when providing an augmented video image based on the video signal.

9. The device of any one of claims 2 and 8, wherein the data processing unit is configured to receive the video signal of the endoscope and to augment the video signal with information from the model of the anatomy of interest when providing an augmented video image based on the video signal.

10. The device of any one of claims 3, 8 and 9, wherein a deep neural net is applied when processing the video signal of the endoscope.

11. The device of any one of claims 8 to 10, further comprising a display for visualizing the augmented video image.

12. A computer program product executable on a data processing unit of a device for assisting in endoscopic surgery, the computer program product comprising sets of instructions which when executed by the data processing unit causing the device
to receive an X-ray image of a trocar and of an anatomy of interest, wherein a trocar holder is configured to arrange the trocar in a fixed relation to the anatomy of interest, while the anatomy is moving,
to receive a model of the anatomy of interest, and
to determine a 3D position and orientation of the trocar relative to the anatomy of interest based on the received X-ray image and on the received model of the anatomy of interest.

13. The computer program product of claim 12, comprising sets of instructions which when executed by the data processing unit causing the device to receive a video signal from an endoscope, wherein the endoscope is configured to be arranged within the trocar, wherein determining the 3D position and orientation of the trocar relative to the anatomy of interest includes determining a 3D position and orientation of the endoscope relative to the anatomy of interest based on the video signal provided by the endoscope.

14. The computer program product of claim 13, comprising sets of instructions which when executed by the data processing unit causing the device to provide an augmented video image based on the video signal.

15. A computer readable medium having stored the computer program product according to any one of claims 12 to 14.
